# EUROPEAN PATENT APPLICATION

(11) **EP 1 790 220 A1**
(43) Date of publication of application: **30.05.2007**
(21) Application number: 05783443.4
(22) Date of filing: 13.09.2005
(51) Int. Cl.: A01K 67/027, A61K 45/00, A61P 1/16, G01N 33/15, G01N 33/50

(54) **PATHOLOGIC MODEL ANIMAL FOR NON-ALCOHOLIC FATTY HEPATITIS**

(30) Priority: 17.09.2004 JP 2004272313
(71) Applicant: BANYU PHARMACEUTICAL CO., LTD., Tokyo 102-8667 (JP)
(72) Inventor: SUZUKI, Jun, Tsukuba Res. Inst. BANYU PHAR.CO LTD., Tsukuba-shi, Ibaraki 300-2611 (JP); IWAASA, Hisashi, Tsukuba Research Institute, Tsukuba-shi, Ibaraki 300-2611 (JP); SASAKI, Minoru, Tsukuba Research Institute, Tsukuba-shi, Ibaraki 300-2611 (JP); ITO, Makoto, Tsukuba Research Institute, Tsukuba-shi, Ibaraki 300-2611 (JP); KANATANI, Akio, BANYU PHARMACEUTICAL CO. LTD., Tsukuba-shi, Ibaraki 3002611 (JP)
(74) Representative: Man, Jocelyn
(86) International application number: PCT/JP2005/016854
(87) International publication number: WO 2006/030792

(57) **Abstract**

An object of the present invention is to provide a pathologic model animal more accurately reflecting the pathologic conditions of nonalcoholic steatohepatitis patients, and the present invention provides the pathologic model animal for nonalcoholic steatohepatitis produced by continuously administering a tetracycline antibiotic to an animal having its body weight significantly increased compared with that of a group fed with a normal diet by feeding it with a high-fat diet.

## Description

### Technical Field

The present invention relates to a pathologic model animal for nonalcoholic steatohepatitis, a method of producing the same and a method of screening a drug using the animal.

### Background Art

Conventionally, it was considered that nonalcoholic fatty liver (fatty liver which is not attributable to alcohol intake) does not have an adverse effect on the body and is a condition which does not need to be treated. However, it has come to be understood recently that some nonalcoholic fatty liver develops into hepatitis due to stimulation such as oxidative stress, and further causes hepatic fibrosis to develop into hepatic cirrhosis or liver cancer. Currently, hepatitis developed from nonalcoholic fatty liver is called nonalcoholic steatohepatitis (NASH), and is considered to be a disease to be treated.

In a screening test or a pharmacological test performed for the purpose of development of a therapeutic agent for nonalcoholic steatohepatitis, it is preferred to use not a normal animal but a pathologic model animal with pathologic conditions of nonalcoholic steatohepatitis patients as many as possible. As such a pathologic model animal, a mouse produced by feeding it with an MCD (methionine-and choline-deficient) diet (hereinafter referred to as an "MCD model") is currently known (Non-patent document 1).

The MCD model is considered to be an excellent pathologic model animal for nonalcoholic steatohepatitis in terms of the point that typical observations of nonalcoholic steatohepatitis, i.e., the onset of steatohepatitis, an increase in alanine aminotransferase (ALT) and aspartate aminotransferase (AST) in the blood, an increase in triglyceride (TG) in the liver, and an increase in mRNA of inflammatory cytokines (such as tumor necrosis factor α (TNF-α) and interleukin-1β (IL-1β)) in the liver are observed.
Non-patent document 1: Rinella, M. E. et al., J. Hepatology, 40: 47-51 (2004)

### Disclosure of the invention

### Problems that the Invention is to Solve

However, while in many nonalcoholic steatohepatitis patients, the body weight, blood insulin level and blood glucose level are normal or higher than the normal value, in the MCD model, these are all lower than the normal value. Therefore, the MCD model does not accurately reflect the actual pathologic conditions of nonalcoholic steatohepatitis patients from this viewpoint.

Thus, an object of the present invention is to solve the above problem of the MCD model and to provide a pathologic model animal more accurately reflecting the actual pathologic conditions of nonalcoholic steatohepatitis patients, a method of producing the same and a method of screening a more effective drug for development of a therapeutic agent for nonalcoholic steatohepatitis.

### Means for Solving the Problems

In order to achieve the above object, the present invention provides a pathologic model animal for nonalcoholic steatohepatitis produced by continuously administering a tetracycline antibiotic to an animal having its body weight significantly increased compared with that of a group fed with a normal diet by feeding it with a high-fat diet.

Such a pathologic model animal for nonalcoholic steatohepatitis is to be a pathologic model animal more accurately reflecting the actual pathologic conditions of nonalcoholic steatohepatitis patients in terms of the point that the body weight, blood insulin level and blood glucose level are normal or higher than the normal value.

As the high-fat diet, a diet which contains at least a protein, a carbohydrate and a fat and in which the calorie derived from the fat accounts for 30% or more of the total calories of the diet is preferred. Further, it is preferred that the tetracycline antibiotic is continuously administered until the blood ALT level, blood AST level, hepatic TG level, hepatic TNF-α mRNA level and hepatic IL-1β mRNA level become significantly higher than those of a normal animal of the same type.

As the pathologic model animal for nonalcoholic steatohepatitis, an animal produced by continuously feeding it with a high-fat diet which contains at least a protein, a carbohydrate and a fat and in which the calorie derived from the fat accounts for 30% or more of the total calories of the diet, until its blood ALT level, blood AST level, hepatic TG level, hepatic TNF-α mRNA level and hepatic IL-1β mRNA level become significantly higher than those of a normal animal of the same type can also be adopted. The thus produced pathologic model animal is also to be a model animal more accurately reflecting the actual pathologic conditions of nonalcoholic steatohepatitis patients in terms of the point that the body weight, blood insulin level and blood glucose level are normal or higher than the normal value. Further, depending on the feeding period of the high-fat diet, such an animal is to be a pathologic model animal for advanced nonalcoholic steatohepatitis involved in hepatic fibrosis.

As described above, the pathologic model animal for nonalcoholic steatohepatitis can be produced by continuously administering a tetracycline antibiotic to an animal having its body weight significantly increased compared with that of a group fed with a normal diet by feeding it with a high-fat diet. In this case, as the high-fat diet, a diet which contains at least a protein, a carbohydrate and a fat and in which the calorie derived from the fat accounts for 30% or more of the total calories of the diet is preferred, and further, it is preferred that the tetracycline antibiotic is continuously administered until the blood ALT level, blood AST level, hepatic TG level, hepatic TNF-α mRNA level and hepatic IL-1β mRNA level become significantly higher than those of a normal animal of the same type.

### Advantage of the Invention

According to the present invention, it becomes possible to solve the problem of the MCD model and to provide a pathologic model animal more accurately reflecting the actual pathologic conditions of nonalcoholic steatohepatitis patients, a method of producing the same and a method of screening a more effective drug for development of a therapeutic agent for nonalcoholic steatohepatitis.

### Brief Description of the Drawings

[Fig. 1] Fig. 1 is a line graph showing the change in the body weight of mice intraperitoneally administered with tetracycline hydrochloride for 10 days after feeding them with HFD or a control diet for 8 weeks.
[Fig. 2] Fig. 2 is a bar graph showing the plasma ALT level in mice intraperitoneally administered with tetracycline hydrochloride for 10 days after feeding them with HFD or a control diet for 8 weeks.
[Fig. 3] Fig. 3 is a bar graph showing the plasma AST level in mice intraperitoneally administered with tetracycline hydrochloride for 10 days after feeding them with HFD or a control diet for 8 weeks.
[Fig. 4] Fig. 4 is a bar graph showing the plasma insulin level in mice intraperitoneally administered with tetracycline hydrochloride for 10 days after feeding them with HFD or a control diet for 8 weeks.
[Fig. 5] Fig. 5 is a bar graph showing the plasma glucose level in mice intraperitoneally administered with tetracycline hydrochloride for 10 days after feeding them with HFD or a control diet for 8 weeks.
[Fig. 6] Fig. 6 is a bar graph showing the hepatic TG level in mice intraperitoneally administered with tetracycline hydrochloride for 10 days after feeding them with HFD or a control diet for 8 weeks.
[Fig. 7] Fig. 7 is a bar graph showing the hepatic TNF-α mRNA level in mice intraperitoneally administered with tetracycline hydrochloride for 10 days after feeding them with HFD or a control diet for 8 weeks.
[Fig. 8] Fig. 8 is a bar graph showing the hepatic IL-1β mRNA level in mice intraperitoneally administered with tetracycline hydrochloride for 10 days after feeding them with HFD or a control diet for 8 weeks.
[Fig. 9] Fig. 9 is a view corresponding to an optical microscopic photograph showing the results of hematoxylin-eosin staining of a section of the middle lobe of the liver of mice intraperitoneally administered with tetracycline hydrochloride at a dose of 30 mg/kg/day for 10 days after feeding them with HFD for 8 weeks.
[Fig. 10] Fig. 10 is a view corresponding to an optical microscopic photograph of an enlarged portion surrounded by a black border in Fig. 9.
[Fig. 11] Fig. 11 is a view corresponding to an optical microscopic photograph showing the results of oil red O staining of a section of the middle lobe of the liver of mice intraperitoneally administered with tetracycline hydrochloride at a dose of 30 mg/kg/day for 10 days after feeding them with HFD for 8 weeks.
[Fig. 12] Fig. 12 is a line graph showing the change in the body weight of mice subcutaneously administered with tetracycline hydrochloride for 10 days after feeding them with HFD for 8 weeks.
[Fig. 13] Fig. 13 is a bar graph showing the plasma ALT level in mice subcutaneously administered with tetracycline hydrochloride for 10 days after feeding them with HFD for 8 weeks.
[Fig. 14] Fig. 14 is a bar graph showing the plasma AST level in mice subcutaneously administered with tetracycline hydrochloride for 10 days after feeding them with HFD for 8 weeks.
[Fig. 15] Fig. 15 is a bar graph showing the plasma insulin level in mice subcutaneously administered with tetracycline hydrochloride for 10 days after feeding them with HFD for 8 weeks.
[Fig. 16] Fig. 16 is a bar graph showing the plasma glucose level in mice subcutaneously administered with tetracycline hydrochloride for 10 days after feeding them with HFD for 8 weeks.
[Fig. 17] Fig. 17 is a bar graph showing the hepatic TG level in mice subcutaneously administered with tetracycline hydrochloride for 10 days after feeding them with HFD for 8 weeks.
[Fig. 18] Fig. 18 is a bar graph showing the hepatic TNF-α mRNA level in mice subcutaneously administered with tetracycline hydrochloride for 10 days after feeding them with HFD for 8 weeks.
[Fig. 19] Fig. 19 is a bar graph showing the hepatic IL-1β mRNA level in mice subcutaneously administered with tetracycline hydrochloride for 10 days after feeding them with HFD for 8 weeks.
[Fig. 20] Fig. 20 is a bar graph showing the body weight of mice fed with HFD or a control diet for 50 weeks.
[Fig. 21] Fig. 21 is a bar graph showing the plasma ALT level in mice fed with HFD or a control diet for 50 weeks.
[Fig. 22] Fig. 22 is a bar graph showing the plasma AST level in mice fed with HFD or a control diet for 50 weeks.
[Fig. 23] Fig. 23 is a bar graph showing the plasma insulin level in mice fed with HFD or a control diet for 50 weeks.
[Fig. 24] Fig. 24 is a bar graph showing the plasma glucose level in mice fed with HFD or a control diet for 50 weeks.
[Fig. 25] Fig. 25 is a bar graph showing the hepatic TG level in mice fed with HFD or a control diet for 50 weeks.
[Fig. 26] Fig. 26 is a bar graph showing the hepatic TNF-α mRNA level in mice fed with HFD or a control diet for 50 weeks.
[Fig. 27] Fig. 27 is a bar graph showing the hepatic IL-1β mRNA level in mice fed with HFD or a control diet for 50 weeks.
[Fig. 28] Fig. 28 is a view corresponding to an optical microscopic photograph showing the results of hematoxylin-eosin staining of a section of the middle lobe of the liver of mice fed with HFD for 50 weeks.
[Fig. 29] Fig. 29 is a view corresponding to an optical microscopic photograph showing the results of hematoxylin-eosin staining of a section of the middle lobe of the liver of mice fed with a control diet for 50 weeks.
[Fig. 30] Fig. 30 is a bar graph showing the hepatic collagen type I (α1) mRNA level in mice fed with HFD or a control diet for 50 weeks.
[Fig. 31] Fig. 31 is a bar graph showing the hepatic α-SMA mRNA level in mice fed with HFD or a control diet for 50 weeks.

### Best Mode for Carrying Out the Invention

Hereinafter, a preferred embodiment of the present invention will be described.

Examples of an animal to be used for producing a pathologic model animal for nonalcoholic steatohepatitis (hereinafter referred to as "NASH pathologic model animal") of the present invention include rats, mice, guinea pigs, hamsters, rabbits, dogs, monkeys and the like, which are generally used as a pathologic model animal for a human disease, and among these, mice are preferred. In male animals, a change in the hormone level due to the estrous cycle does not occur and a variation in the pathologic conditions can be suppressed, therefore, the animal is preferably a male animal.

In order to produce the NASH pathologic model animal, first, by feeding such an animal with a high-fat diet, its body weight is significantly increased compared with that of a group fed with a normal diet. The high-fat diet to be fed is preferably a high-fat diet which contains at least a protein, a carbohydrate and a fat and in which the calorie derived from the fat accounts for 30% or more of the total calories. The lower limit of the proportion of the calorie derived from the fat is preferably 45%, more preferably 60%. On the other hand, the upper limit thereof is preferably 80%, more preferably 70%.

Incidentally, whether or not its body weight is significantly increased compared with that of a group fed with a normal diet by feeding it with a high-fat diet can be determined by a t-test. Here, a significance level in the t-test is 1% or 5%, preferably 1%.

The NASH pathologic model animal is produced by continuously administering a tetracycline antibiotic to an animal having its body weight significantly increased as described above. Here, the phrase "continuously administering a tetracycline antibiotic" means administering a tetracycline antibiotic a plurality of times, preferably at regular intervals. Examples of the tetracycline antibiotic to be continuously administered include tetracycline (TC), oxytetracycline (OTC), methacycline (MTC), doxycycline (DOXY), minocycline (MINO) and salts thereof (such as hydrochlorides thereof).

It is preferred that the administration of a tetracycline antibiotic is continued until the blood ALT level, blood AST level, hepatic TG level, hepatic TNF-α mRNA level and hepatic IL-1β mRNA level become significantly higher than those of a normal animal of the same type. Further, it is more preferred that the administration thereof is continued until the blood ALT level, blood AST level, hepatic TG level, hepatic TNF-α mRNA level and hepatic IL-1β mRNA level become significantly higher than those of a normal animal of the same type, and the presence of fatty liver and the invasion of inflammatory cells are observed in a histopathological test of liver. Incidentally, whether or not the above values are significantly higher than those of a normal animal of the same type can be determined by a t-test, in which a significance level is set to 1% or 5% (preferably 1%).

Incidentally, the NASH pathologic model animal can be produced by continuously feeding an animal with a high-fat diet which contains at least a protein, a carbohydrate and a fat and in which the calorie derived from the fat accounts for 30% or more of the total calories of the diet for a certain period of time until its blood ALT level, blood AST level, hepatic TG level, hepatic TNF-α mRNA level and hepatic IL-1β mRNA level become significantly higher than those of a normal animal of the same type without continuously administering a tetracycline antibiotic. Here, the phrase "continuously feeding an animal" means feeding an animal a plurality of times.

In the case where the NASH pathologic model animal is produced by administering a tetracycline antibiotic after feeding an animal with a high-fat diet, the feeding period of high-fat diet before administering the tetracycline antibiotic may be a period which is sufficient to form fatty liver. For example, in the case of mice, it is preferably 6 to 10 weeks, more preferably 7 to 9 weeks. The high-fat diet is preferably fed also during the period of administering the tetracycline antibiotic.

In the case where the NASH pathologic model animal is produced by administering a tetracycline antibiotic after feeding an animal with a high-fat diet, the dose and administration period of tetracycline antibiotic is preferably 20 to 100 mg/kg/day for 7 to 14 days, more preferably 20 to 40 mg/kg/day for 9 to 11 days in mice. In the case of a dose of 20 mg/kg/day for less than 7 days, fatty liver may not develop into hepatitis in some cases. On the other hand, in the case of a dose of 100 mg/kg/day for more than 15 days, there is a possibility that any of the body weight, blood insulin level and blood glucose level may become lower than a normal value. The administration method of tetracycline antibiotic is preferably intraperitoneal administration or subcutaneous administration. Here, the tetracycline antibiotic may be administered in the form of a pharmacologically acceptable salt thereof.

In the case where the NASH pathologic model animal is produced by continuously feeding an animal with a high-fat diet, the feeding period of high-fat diet may be a period which is sufficient to develop steatohepatitis. In the case of mice, it is preferably 20 weeks or more, more preferably 40 weeks or more. In the case of less than 20 weeks, there is a possibility that the onset of steatohepatitis does not sufficiently occur. Further, in order to produce the NASH pathologic model animal with hepatic fibrosis, in the case of mice, it is preferred to feed mice with a high-fat diet for 40 weeks or more. The occurrence of hepatic fibrosis can be confirmed by, for example, examining that the hepatic collagen type I (α1) mRNA level or the hepatic α-SMA (smooth muscle actin) mRNA level is significantly higher than that of a normal animal of the same type. Here, the "collagen type I (α1)" represents the α1-chain of collagen type I.

By using the above-mentioned NASH pathologic model animal, it becomes possible to screen a drug for nonalcoholic steatohepatitis. That is, a method of screening a drug for nonalcoholic steatohepatitis comprising the step of administering a candidate drug for nonalcoholic steatohepatitis to the NASH pathologic model animal is provided. In this screening method, after the step of administering a candidate drug, the step of determining whether or not the candidate drug is effective as a drug for nonalcoholic steatohepatitis can be provided. In this step, for example, by examining that the blood ALT level, blood AST level, hepatic TG level, hepatic TNF-α mRNA level and hepatic IL-1β mRNA level are not significantly higher than those of a normal animal of the same type, the effectiveness thereof as a drug for nonalcoholic steatohepatitis can be determined. Further, by examining that the invasion of inflammatory cells is not observed in a histopathological test of liver, the effectiveness thereof as a drug for nonalcoholic steatohepatitis can also be determined.

The NASH pathologic model animal of the present invention and the method of screening a drug using the same can be used in the same manner as a known pathologic model animal and a method of screening a drug using the same in a screening test or a pharmacological test for the purpose of development of a pharmaceutical product.

### Examples

Hereinafter, the present invention will be more specifically described with reference to Examples and Comparative examples. However, the invention is not limited to the following Examples.

In the following Examples and Comparative examples, as an animal for producing a pathologic model animal, C57BL/6J male mice (Nippon CLEA, Inc.) were used. Further, as a high-fat diet (hereinafter referred to as "HFD"), D12492 (fat content: 60 kcal%) (Research Diet Inc.) was used. As a control diet, CE-2 (Nippon CLEA, Inc.) to be used for normal rearing of mice was used. As a tetracycline antibiotic, tetracycline hydrochloride (Sigma Inc.) was used. The administration of tetracycline hydrochloride was carried out by injection of a solution of 0, 10, 30 or 100 mg/10 mL of tetracycline hydrochloride prepared with a 0.5% physiological saline solution. Here, the "solution of 0 mg/10 mL of tetracycline hydrochloride" means a 0.5% physiological saline solution.

### (Example 1 and Comparative example 1)

29 mice (6 weeks of age) were divided into a group of 15 mice fed with HFD (hereinafter referred to as an "HFD group") and a group of 14 mice fed with a control diet (hereinafter referred to as a "control group"). The following experiments related to the HFD group (15 mice) and the control group (14 mice) were assigned to Example 1 and Comparative example 1, respectively.

### Feeding of HFD or control diet and intraperitoneal administration of tetracycline hydrochloride

First, the mice in the HFD group and the control group were fed ad libitum with HFD and the control diet for 8 weeks, respectively. Then, the mice in the HFD group and the control group were divided into 3 groups, respectively, in such a manner that the body weight and the body fat percentage were equal in the respective groups, thereby to form 3 groups in the HFD group (5 mice x 3 groups), and 3 groups in the control group (5 mice x 2 groups and 4 mice x 1 group). Then, tetracycline hydrochloride was intraperitoneally administered to both the HFD group and the control group at a dose of 0, 10 and 30 mg/kg/day for 10 days (hereinafter, the groups administered with tetracycline hydrochloride at a dose of 0, 10 and 30 mg/kg/day are referred to as "TC 0 group", "TC 10 group" and "TC 30 group", respectively). The mice were fed ad libitum with HFD or the control diet also during the period of administering tetracycline hydrochloride.

### Measurement of body weight

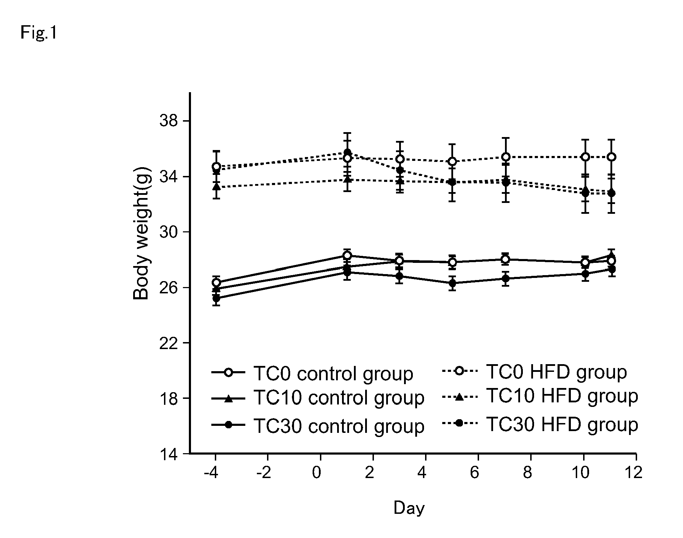

The body weight of mice was measured on 4 days before initiation of administration of tetracycline hydrochloride and on day 1, 3, 5, 7, 10 and 11 after initiation of administration thereof. Fig. 1 is a line graph showing the change in the body weight of mice intraperitoneally administered with tetracycline hydrochloride for 10 days after feeding them with HFD or the control diet for 8 weeks. From Fig. 1, it is found that the mice in the HFD group showed a body weight significantly higher than that of the normal mice (mice in the TC 0 group among the control groups) at the time of initiation of administration of tetracycline hydrochloride, and the value was substantially maintained during the period of administration thereof. Incidentally, in Fig. 1, the term "days" represents the number of days elapsed after initiation of administration of tetracycline hydrochloride.

### Blood collection and dissection

On the next day after completion of administration of tetracycline hydrochloride (on day 11 after initiation of administration), the mice were anesthetized by isoflurane inhalation. After it was confirmed that the mice lost pain sensation by pinching the tail with forceps, the mice underwent laparotomy and the blood was collected from the abdominal vena cava with a syringe containing heparin. The collected blood was centrifuged at 4°C for 10 minutes at 7500 rpm thereby to separate plasma. The obtained plasma was cryopreserved. After the blood collection, the thorax was immediately opened thereby to achieve complete exsanguination, and then, the liver was excised. One lobe of the liver was cryopreserved. Further, a portion (about 50 mg) of the liver was excised and cryopreserved in liquid nitrogen. Further, the middle lobe of the liver was fixed in a 10% neutral buffered formalin solution.

### Measurement of plasma ALT level, plasma AST level, plasma insulin level and plasma glucose level

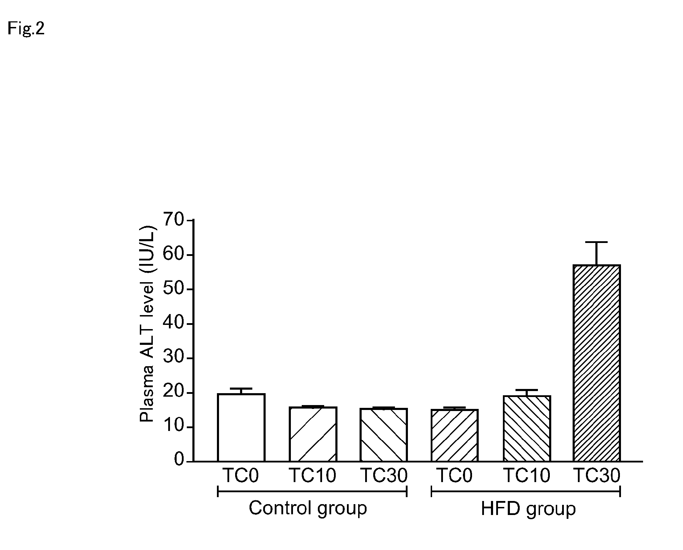

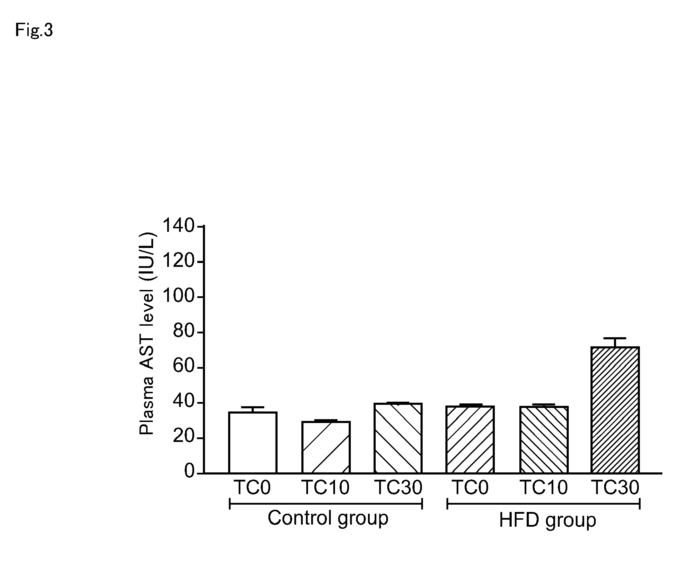

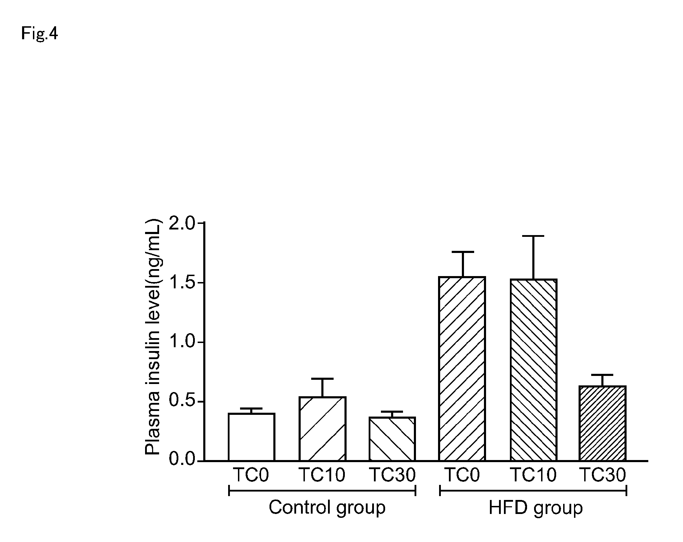

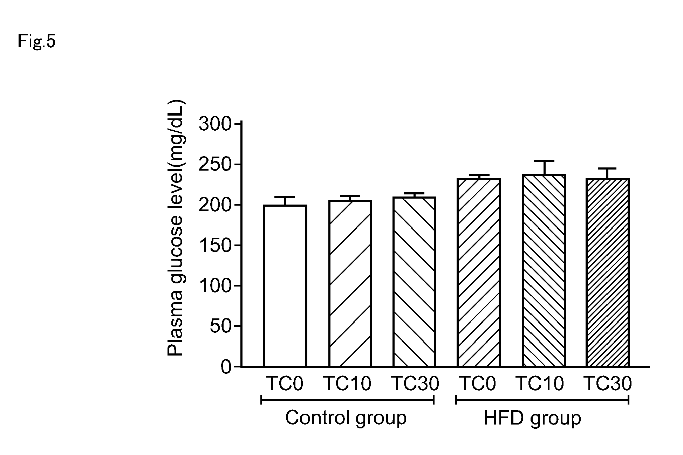

By using the cryopreserved plasma, the plasma ALT level, plasma AST level and plasma glucose level were measured using Hitachi autoanalyzer 7070 (Hitachi Hi Technologies), and the plasma insulin level was measured using an insulin assay kit (Seikagaku Corporation). Fig. 2 is a bar graph showing the plasma ALT level in mice intraperitoneally administered with tetracycline hydrochloride for 10 days after feeding them with HFD or the control diet for 8 weeks. Fig. 3 is a bar graph showing the plasma AST level in mice intraperitoneally administered with tetracycline hydrochloride for 10 days after feeding them with HFD or the control diet for 8 weeks. From Figs. 2 and 3, it is found that among the HFD groups, the mice in the TC 30 group showed a plasma ALT level and a plasma AST level significantly higher than those of the normal mice. Fig. 4 is a bar graph showing the plasma insulin level in mice intraperitoneally administered with tetracycline hydrochloride for 10 days after feeding them with HFD or the control diet for 8 weeks. Fig. 5 is a bar graph showing the plasma glucose level in mice intraperitoneally administered with tetracycline hydrochloride for 10 days after feeding them with HFD or the control diet for 8 weeks. From Figs. 4 and 5, it is found that the mice in the HFD group showed a plasma insulin level and a plasma glucose level equivalent to or higher than those of the normal mice.

### Measurement of hepatic TG level

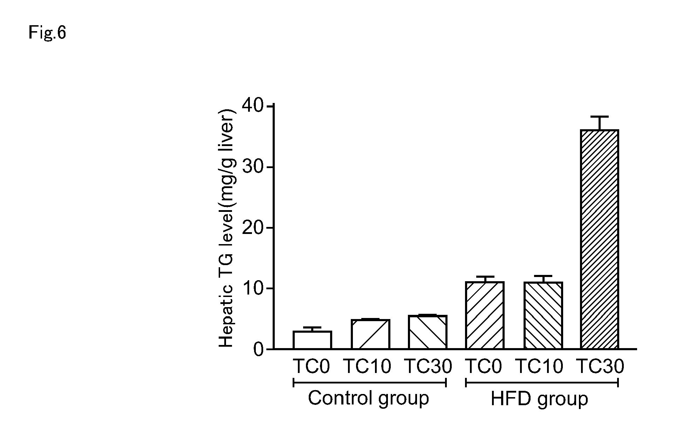

The cryopreserved one lobe of the liver was homogenized, and a fat fraction was extracted from a portion of this homogenate with Folch reagent. Then, the extracted fat fraction was dried and hardened with nitrogen gas, and the TG level was measured using Determiner L TG II (Kyowa Medex). Fig. 6 is a bar graph showing the hepatic TG level in mice intraperitoneally administered with tetracycline hydrochloride for 10 days after feeding them with HFD or the control diet for 8 weeks. From Fig. 6, it is found that the mice in the HFD group showed a hepatic TG level significantly higher than that of the normal mice, and in particular, the mice in the TC 30 group showed a considerably high hepatic TG level.

### Measurement of hepatic TNF-α mRNA level and hepatic IL-1β mRNA level

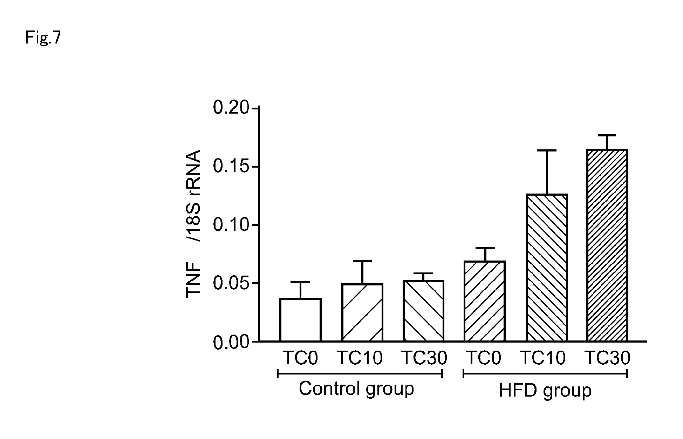

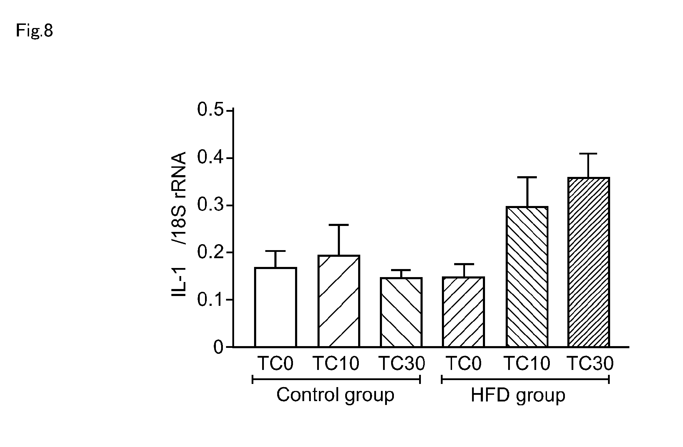

From the cryopreserved portion of the liver in liquid nitrogen, RNA was extracted using ISOGEN (Nippon Gene Co. Ltd.), and cDNA was synthesized using a real-time quantitative PCR reagent available from Applied Biosystems Inc. Then, by using 18S rRNA as an internal standard, the TNF-α mRNA level and IL-1β mRNA level were measured using ABI PRISM 7900 HT Sequence Detection System (Applied Biosystems Inc.). Fig. 7 is a bar graph showing the hepatic TNF-α mRNA level in mice intraperitoneally administered with tetracycline hydrochloride for 10 days after feeding them with HFD or the control diet for 8 weeks. Fig. 8 is a bar graph showing the hepatic IL-1β mRNA level in mice intraperitoneally administered with tetracycline hydrochloride for 10 days after feeding them with HFD or the control diet for 8 weeks. From Figs. 7 and 8, it is found that among the HFD groups, the mice in the TC 10 group and the TC 30 group showed a hepatic TNF-α mRNA level and a hepatic IL-1β mRNA level significantly higher than those of the normal mice.

### Histopathological test of liver

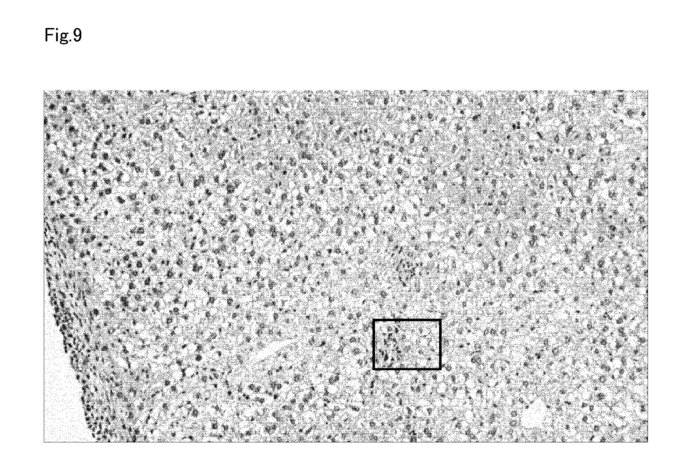

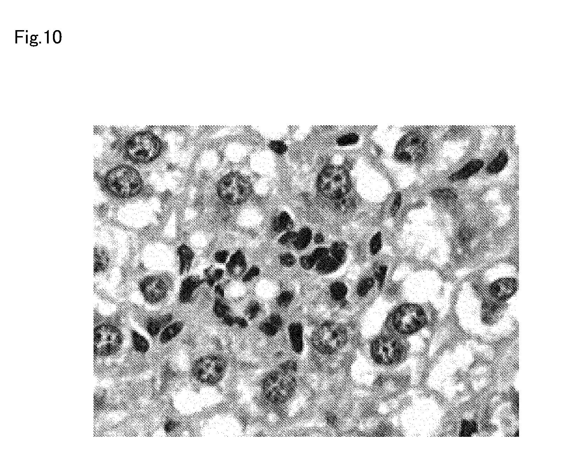

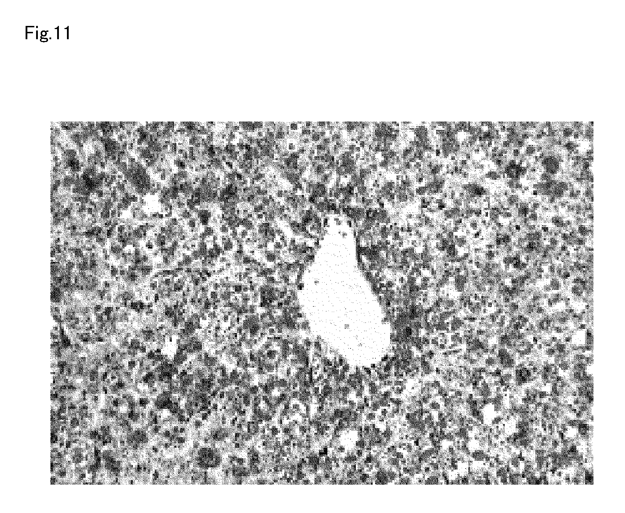

A paraffin section was prepared from the middle lobe of the liver fixed in the formalin solution, and hematoxylin-eosin staining and oil red O staining (fat staining) were carried out, and a histopathological test was carried out. Fig. 9 is a view corresponding to an optical microscopic photograph showing the results of hematoxylin-eosin staining of the section of the middle lobe of the liver of mice intraperitoneally administered with tetracycline hydrochloride at a dose of 30 mg/kg/day for 10 days after feeding them with HFD for 8 weeks. Fig. 10 is a view corresponding to an optical microscopic photograph of an enlarged portion surrounded by a black border in Fig. 9. Fig. 11 is a view corresponding to an optical microscopic photograph showing the results of oil red O staining of the section of the middle lobe of the liver of mice intraperitoneally administered with tetracycline hydrochloride at a dose of 30 mg/kg/day for 10 days after feeding them with HFD for 8 weeks. From Figs. 9 to 11, it is found that the presence of fatty liver and the invasion of inflammatory cells were observed in the mice in the TC 30 group among the HFD groups.

From Example 1 and Comparative example 1, it was shown that by intraperitoneally administering tetracycline hydrochloride to mice at a dose of 30 mg/kg/day for 10 days after feeding them with a high-fat diet for 8 weeks, an NASH pathologic model animal in which the body weight, blood insulin level and blood glucose level are normal or higher than the normal value is produced.

### (Example 2)

### Feeding of HFD and subcutaneous administration of tetracycline hydrochloride

20 mice (7 weeks of age) were fed ad libitum with HFD for 8 weeks. Then, the mice were divided into 4 groups (5 mice x 4 groups) in such a manner that the body weight and the body fat percentage were equal in the respective groups, and subcutaneously administered with tetracycline hydrochloride at a dose of 0, 30, 60 and 100 mg/kg/day for 10 days (hereinafter, the groups administered with tetracycline hydrochloride at a dose of 0, 30, 60 and 100 mg/kg/day are referred to as "TC 0 group", "TC 30 group", "TC 60 group" and "TC 100 group", respectively). The mice were fed ad libitum with HFD also during the period of administering tetracycline hydrochloride.

### Measurement of body weight

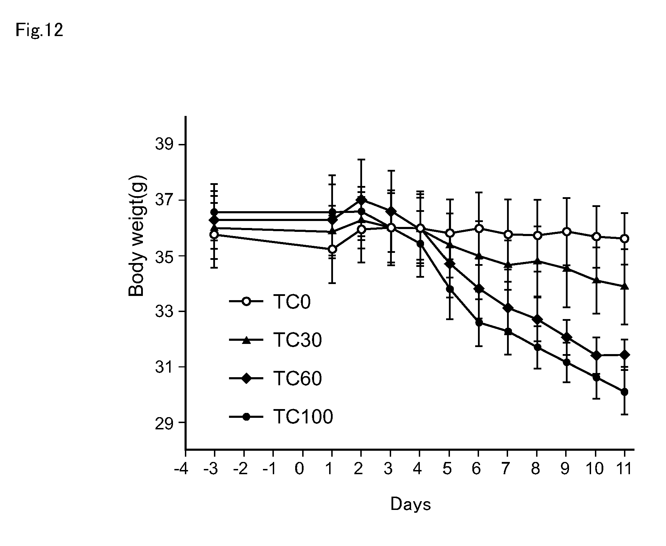

The body weight of mice was measured on 3 days before initiation of administration of tetracycline hydrochloride and everyday from the next day until day 11 after initiation of administration thereof. Fig. 12 is a line graph showing the change in the body weight of mice subcutaneously administered with tetracycline hydrochloride for 10 days after feeding them with HFD for 8 weeks. From Fig. 12, it is found that the mice in any group showed a body weight significantly higher than that of the normal mice at the time of initiation of administration of tetracycline hydrochloride, and the mice in any group also showed a body weight equivalent to or higher than that of the normal mice at the time of completion of administration of tetracycline hydrochloride, although the body weight decreased depending on the dose of tetracycline hydrochloride and the administration period thereof (see Fig. 1). Incidentally, in Fig. 12, the term "days" represents the number of days elapsed after initiation of administration of tetracycline hydrochloride.

### Blood collection and dissection

Blood collection and dissection were carried out in the same manner as in Example 1.

### Measurement of plasma ALT level, plasma AST level, plasma insulin level and plasma glucose level

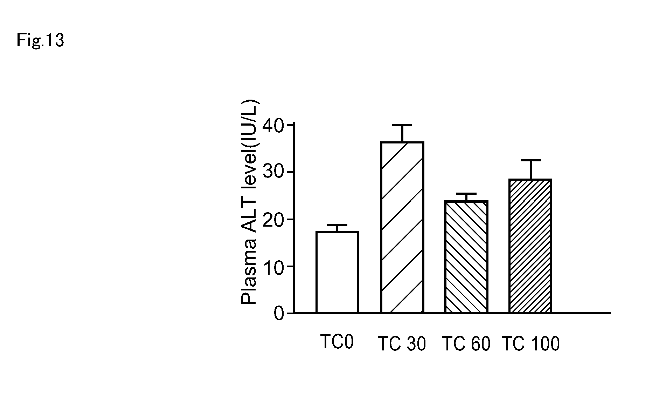

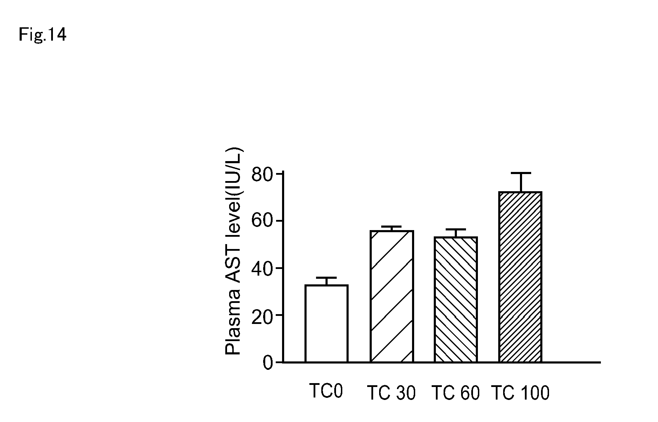

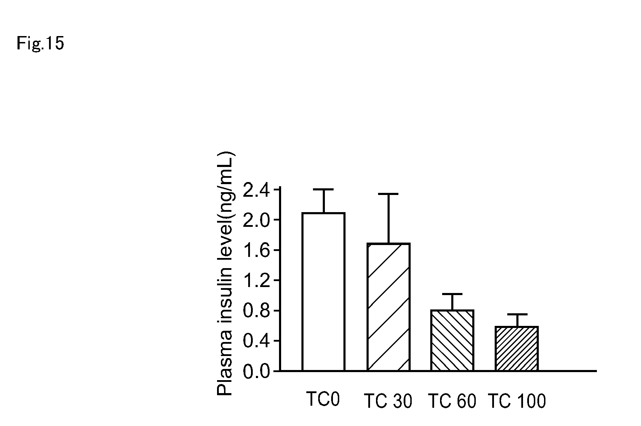

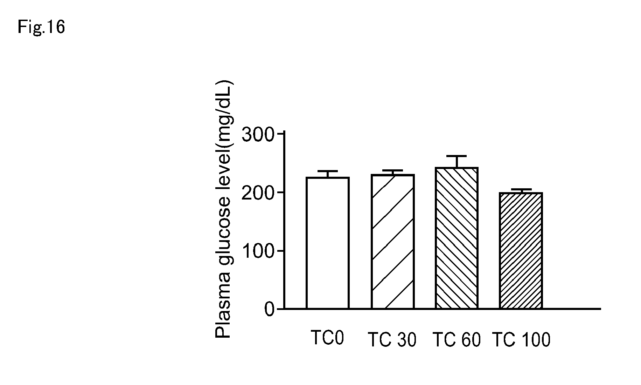

The plasma ALT level, plasma AST level, plasma insulin level and plasma glucose level were measured in the same manner as in Example 1. Fig. 13 is a bar graph showing the plasma ALT level in mice subcutaneously administered with tetracycline hydrochloride for 10 days after feeding them with HFD for 8 weeks. Fig. 14 is a bar graph showing the plasma AST level in mice subcutaneously administered with tetracycline hydrochloride for 10 days after feeding them with HFD for 8 weeks. From Figs. 13 and 14, it is found that the mice in the TC 30 group, TC 60 group and TC 100 group showed a plasma ALT level and a plasma AST level significantly higher than those of the normal mice (see Figs. 2 and 3). Fig. 15 is a bar graph showing the plasma insulin level in mice subcutaneously administered with tetracycline hydrochloride for 10 days after feeding them with HFD for 8 weeks. From Fig. 15, it is found that the mice in the TC 0 group, TC 30 group and TC 60 group showed a plasma insulin level higher than that of the normal mice, and the mice in the TC 100 group showed a plasma insulin level equivalent to that of the normal mice (see Fig. 4). Fig. 16 is a bar graph showing the plasma glucose level in mice subcutaneously administered with tetracycline hydrochloride for 10 days after feeding them with HFD for 8 weeks. From Fig. 16, it is found that the mice in any group showed a plasma glucose level higher than that of the normal mice (see Fig. 5).

### Measurement of hepatic TG level

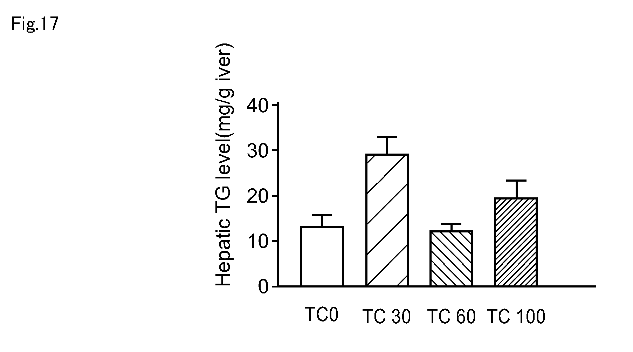

The hepatic TG level was measured in the same manner as in Example 1. Fig. 17 is a bar graph showing the hepatic TG level in mice subcutaneously administered with tetracycline hydrochloride for 10 days after feeding them with HFD for 8 weeks. From Fig. 17, it is found that the mice in any group showed a hepatic TG level significantly higher than that of the normal mice, and in particular, the mice in the TC 30 group showed a considerably high hepatic TG level (see Fig. 6).

### Measurement of hepatic TNF-α mRNA level and hepatic IL-1β mRNA level

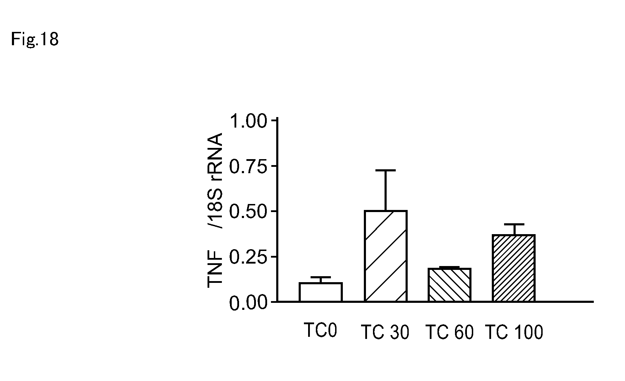

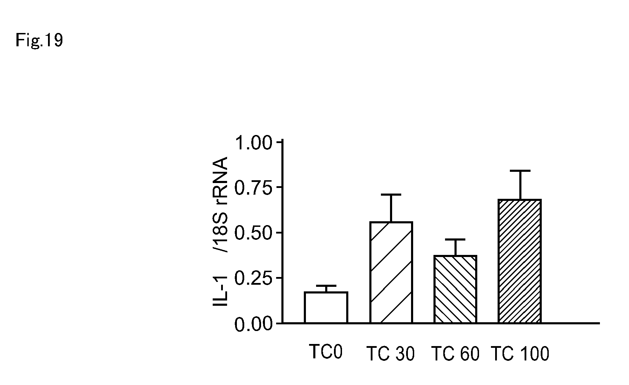

The hepatic TNF-α mRNA level and hepatic IL-1β mRNA level were measured in the same manner as in Example 1. Fig. 18 is a bar graph showing the hepatic TNF-α mRNA level in mice subcutaneously administered with tetracycline hydrochloride for 10 days after feeding them with HFD for 8 weeks. Fig. 19 is a bar graph showing the hepatic IL-1β mRNA level in mice subcutaneously administered with tetracycline hydrochloride for 10 days after feeding them with HFD for 8 weeks. From Figs. 18 and 19, it is found that the mice in the TC 30 group, TC 60 group and TC 100 group showed a hepatic TNF-α mRNA level and a hepatic IL-1β mRNA level significantly higher than those of the normal mice, and in particular, the mice in the TC 30 group showed a considerably high value in both mRNA levels (see Figs. 7 and 8).

### Histopathological test of liver

A histopathological test of liver was carried out in the same manner as in Example 1. The presence of fatty liver and the invasion of inflammatory cells were observed in the mice in the TC 30 group, TC 60 group and TC 100 group.

From Example 2, it was shown that by subcutaneously administering tetracycline hydrochloride to mice for 10 days after feeding them with a high-fat diet for 8 weeks, an NASH pathologic model animal in which the body weight, blood insulin level and blood glucose level are normal or higher than the normal value is produced.

### (Example 3 and Comparative example 2)

12 mice (7 weeks of age) were divided into an HFD group consisting of 6 mice and a control group consisting of 6 mice. The following experiments related to the HFD group (6 mice) and the control group (6 mice) were assigned to Example 3 and Comparative example 2, respectively.

### Feeding of HFD or control diet

The mice in the HFD group and the control group were fed ad libitum with HFD and the control diet for 50 weeks, respectively.

### Measurement of body weight

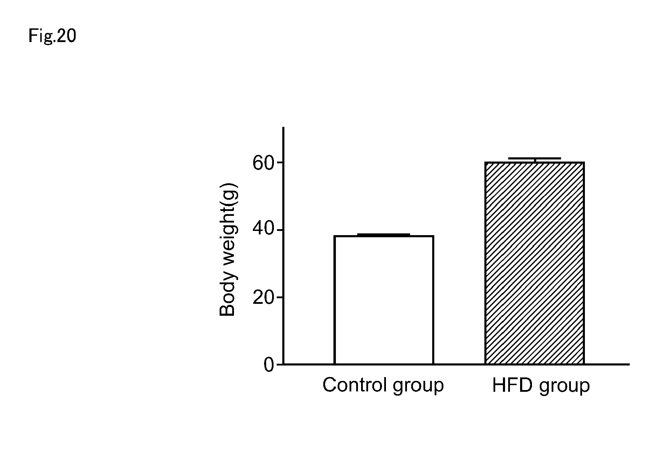

The body weight of mice was measured after completion of feeding of HFD or the control diet and before blood collection (before undergoing anesthesia). Fig. 20 is a bar graph showing the body weight of mice fed with HFD or the control diet for 50 weeks. From Fig. 20, it is found that the mice in the HFD group showed a body weight significantly higher than that of the normal mice (see Fig. 1).

### Blood collection and dissection

Blood collection and dissection were carried out in the same manner as in Example 1 immediately after completion of feeding of HFD or the control diet.

### Measurement of plasma ALT level, plasma AST level, plasma insulin level and plasma glucose level

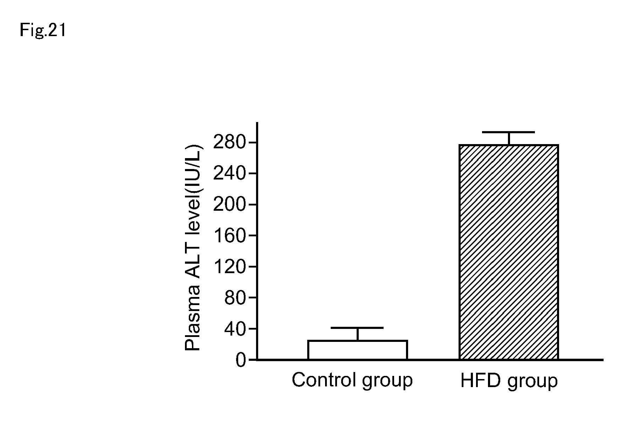

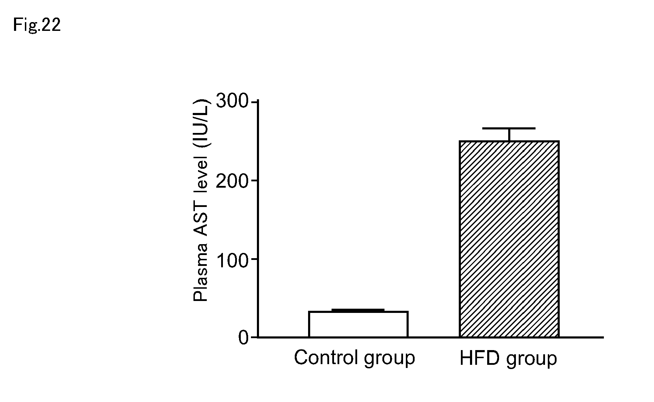

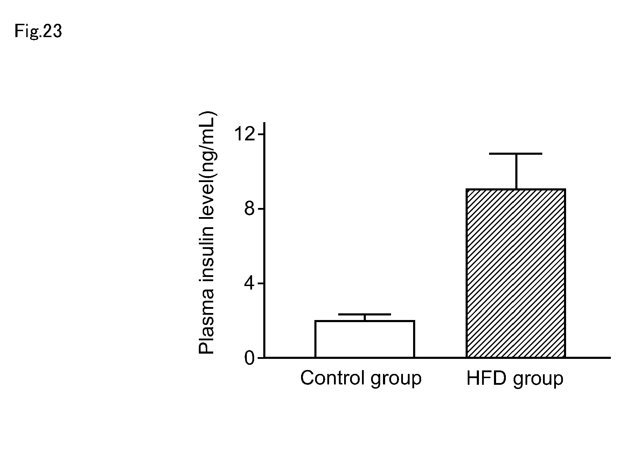

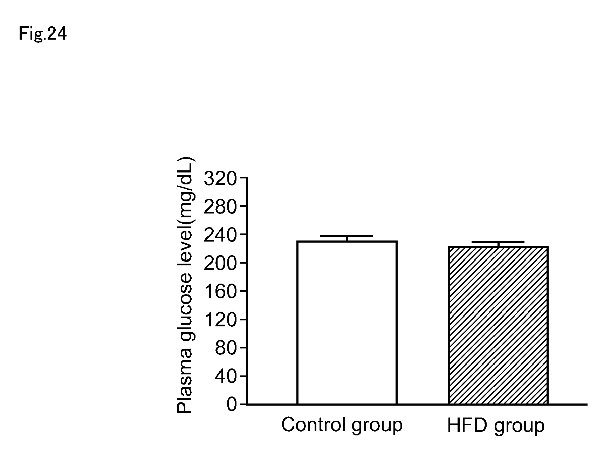

The plasma ALT level, plasma AST level, plasma insulin level and plasma glucose level were measured in the same manner as in Example 1. Fig. 21 is a bar graph showing the plasma ALT level in mice fed with HFD or the control diet for 50 weeks. Fig. 22 is a bar graph showing the plasma AST level in mice fed with HFD or the control diet for 50 weeks. From Figs. 21 and 22, it is found that the mice in the HFD group showed a plasma ALT level and a plasma AST level significantly higher than those of the normal mice (see Figs. 2 and 3). Fig. 23 is a bar graph showing the plasma insulin level in mice fed with HFD or the control diet for 50 weeks. From Fig. 23, it is found that the mice in the HFD group showed a plasma insulin level higher than that of the normal mice (see Fig. 4). Fig. 24 is a bar graph showing the plasma glucose level in mice fed with HFD or the control diet for 50 weeks. From Fig. 24, it is found that the mice in the HFD group showed a plasma glucose level equivalent to that of the normal mice (see Fig. 5).

### Measurement of hepatic TG level

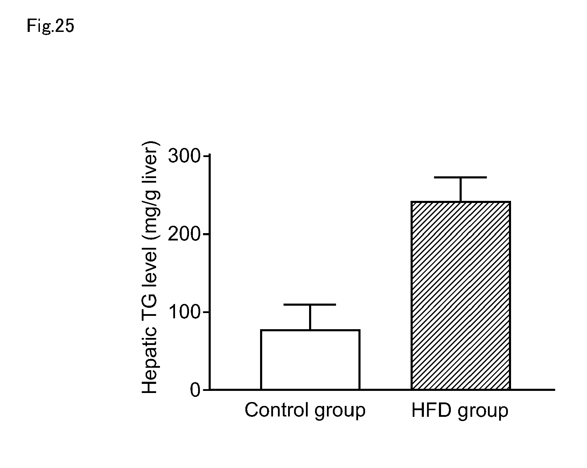

The hepatic TG level was measured in the same manner as in Example 1. Fig. 25 is a bar graph showing the hepatic TG level in mice fed with HFD or the control diet for 50 weeks. From Fig. 25, it is found that the mice in the HFD group showed a hepatic TG level significantly higher than that of the normal mice (see Fig. 6).

### Measurement of hepatic TNF-α mRNA level and hepatic IL-1β mRNA level

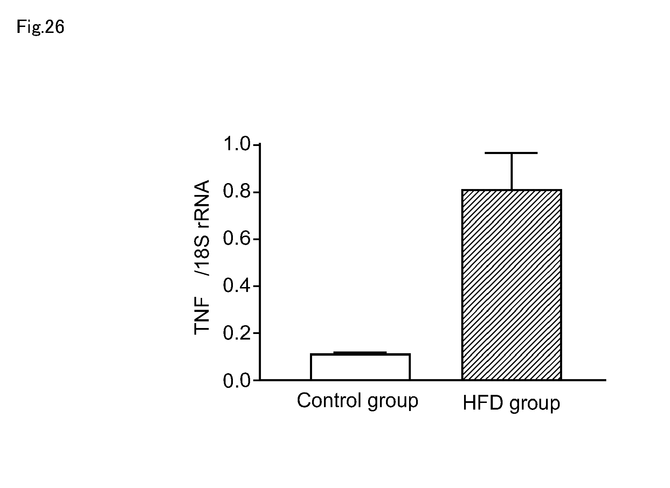

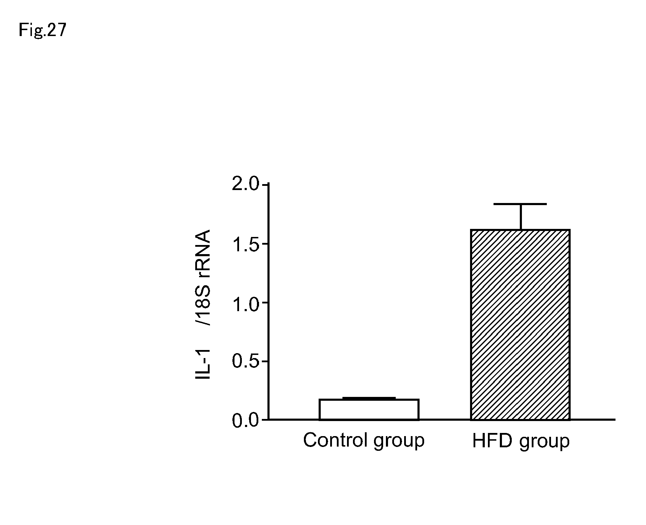

The hepatic TNF-α mRNA level and hepatic IL-1β mRNA level were measured in the same manner as in Example 1. Fig. 26 is a bar graph showing the hepatic TNF-α mRNA level in mice fed with HFD or the control diet for 50 weeks. Fig. 27 is a bar graph showing the hepatic IL-1β mRNA level in mice fed with HFD or the control diet for 50 weeks. From Figs. 26 and 27, it is found that the mice in the HFD group showed a hepatic TNF-α mRNA level and a hepatic IL-1β mRNA level significantly higher than those of the normal mice (see Figs. 7 and 8).

### Histopathological test of liver

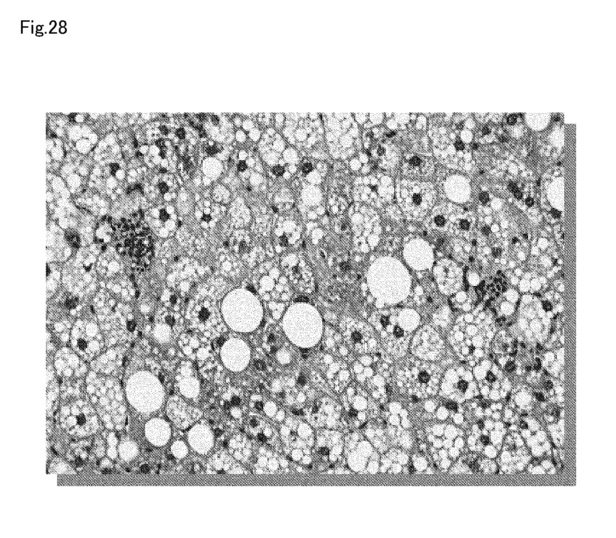

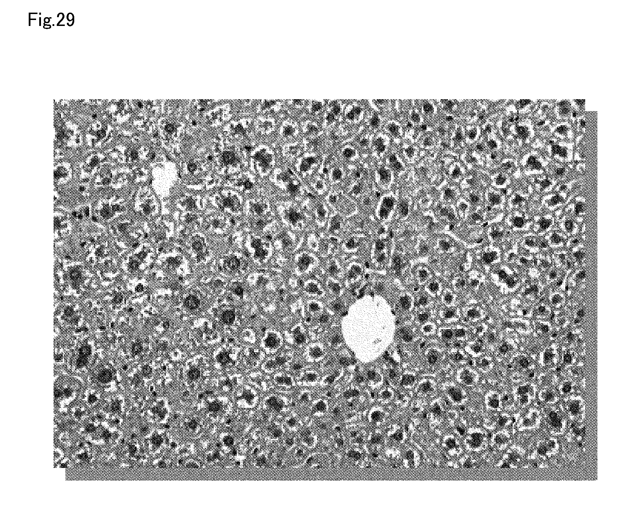

A histopathological test of liver was carried out in the same manner as in Example 1. Fig. 28 is a view corresponding to an optical microscopic photograph showing the results of hematoxylin-eosin staining of a section of the middle lobe of the liver of mice fed with HFD for 50 weeks. Fig. 29 is a view corresponding to an optical microscopic photograph showing the results of hematoxylin-eosin staining of a section of the middle lobe of the liver of mice fed with the control diet for 50 weeks. From Figs. 28 and 29, it is found that the presence of fatty liver and the invasion of inflammatory cells were observed in the mice in the HFD group.

### Measurement of hepatic collagen type I (α1) mRNA level and hepatic α-SMA mRNA level

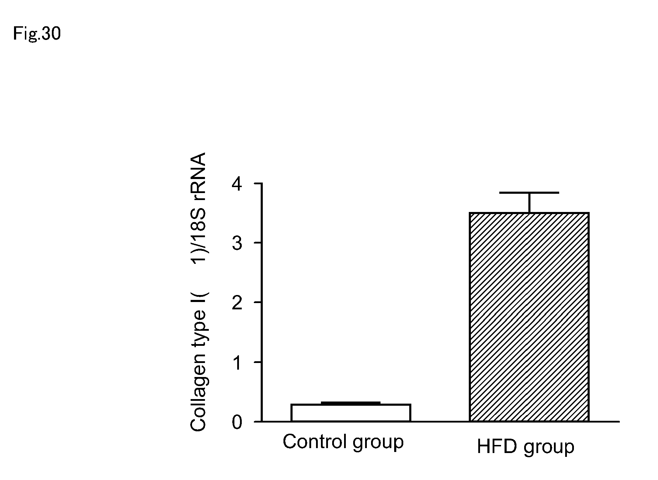

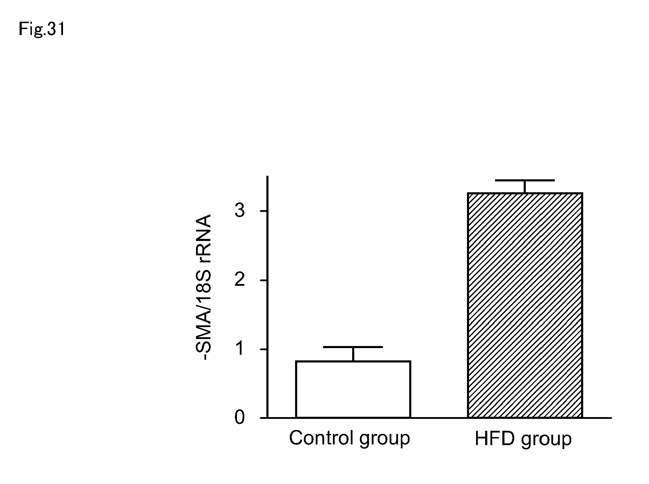

Whether or not hepatic fibrosis occurred was confirmed by using the hepatic collagen type I (α1) mRNA level and the hepatic α-SMA mRNA level as an indicator.
The measurement of the hepatic collagen type I (α1) mRNA level and hepatic α-SMA mRNA level was carried out by the same method as the method of measuring the hepatic TNF-α mRNA level and hepatic IL-1β mRNA level in Example 1. Fig. 30 is a bar graph showing the hepatic collagen type I (α1) mRNA level in mice fed with HFD or the control diet for 50 weeks. Fig. 31 is a bar graph showing the hepatic α-SMA mRNA level in mice fed with HFD or the control diet for 50 weeks. From Figs. 30 and 31, it is found that the mice in the HFD group showed a hepatic collagen type I (α1) mRNA level and a hepatic α-SMA mRNA level significantly higher than those of the normal mice. Further, from the results, it is found that in the mice of the HFD group, hepatic fibrosis occurred unlike the normal mice.

From Example 3 and Comparative example 2, it was shown that by feeding mice with a high-fat diet for 50 weeks, an NASH pathologic model animal in which the body weight, blood insulin level and blood glucose level are normal or higher than the normal value and hepatic fibrosis occurred is produced.

### Industrial Applicability

The present invention can be utilized in the development of a therapeutic agent for nonalcoholic steatohepatitis.

## Claims

1. A pathologic model animal for nonalcoholic steatohepatitis, which is produced by continuously administering a tetracycline antibiotic to an animal having its body weight significantly increased compared with that of a group fed with a normal diet by feeding it with a high-fat diet.

2. The pathologic model animal for nonalcoholic steatohepatitis according to claim 1, wherein the high-fat diet is a high-fat diet which contains at least a protein, a carbohydrate and a fat and in which the calorie derived from the fat accounts for 30% or more of the total calories.

3. The pathologic model animal for nonalcoholic steatohepatitis according to claim 1 or 2, which is produced by continuously administering the tetracycline antibiotic to an animal until its blood ALT level, blood AST level, hepatic TG level, hepatic TNF-α mRNA level and hepatic IL-1β mRNA level become significantly higher than those of a normal animal of the same type.

4. A pathologic model animal for nonalcoholic steatohepatitis, which is produced by continuously feeding it with a high-fat diet which contains at least a protein, a carbohydrate and a fat and in which the calorie derived from the fat accounts for 30% or more of the total calories, until its blood ALT level, blood AST level, hepatic TG level, hepatic TNF-α mRNA level and hepatic IL-1β mRNA level become significantly higher than those of a normal animal of the same type.

5. A method of producing a pathologic model animal for nonalcoholic steatohepatitis comprising continuously administering a tetracycline antibiotic to an animal having its body weight significantly increased compared with that of a group fed with a normal diet by feeding it with a high-fat diet.

6. The method of producing a pathologic model animal for nonalcoholic steatohepatitis according to claim 5, wherein the high-fat diet is a high-fat diet which contains at least a protein, a carbohydrate and a fat and in which the calorie derived from the fat accounts for 30% or more of the total calories.

7. The method of producing a pathologic model animal for nonalcoholic steatohepatitis according to claim 5 or 6, comprising continuously administering the tetracycline antibiotic to an animal until its blood ALT level, blood AST level, hepatic TG level, hepatic TNF-α mRNA level and hepatic IL-1β mRNA level become significantly higher than those of a normal animal of the same type.

8. A method of screening a drug for nonalcoholic steatohepatitis comprising the step of administering a candidate drug to the pathologic model animal for nonalcoholic steatohepatitis according to any one of claims 1 to 4.
